# EUROPEAN PATENT SPECIFICATION

(11) **EP 4 138 102 B1**
(45) Date of publication and mention of the grant of the patent: **26.11.2025**
(21) Application number: 21788037.6
(22) Date of filing: 12.04.2021
(51) Int. Cl.: H01G 4/35, H01G 4/228, H01G 4/236, H01G 4/224, H01G 2/10, H01G 4/242, H01G 9/10, A61N 1/375

(54) **FEEDTHROUGH CAPACITOR NOT PACKAGED BY GLASS AND APPLICABLE TO AIRTIGHT DEVICE**
NICHT GLASVERPACKTER DURCHFÜHRUNGSKONDENSATOR FÜR LUFTDICHTE VORRICHTUNG
CONDENSATEUR DE TRAVERSÉE NON ENCAPSULÉ PAR DU VERRE ET APPLICABLE À UN DISPOSITIF ÉTANCHE À L'AIR

(30) Priority: 14.04.2020 CN 202020543329 U
(43) Date of publication of application: 22.02.2023
(73) Proprietor: Fujian Ouzhong Electronics Co., Ltd, Fuzhou, Fujian 350008 (CN)
(72) Inventor: ZHENG, Chaoyong, Fuzhou, Fujian 350008 (CN); YE, Bin, Fuzhou, Fujian 350008 (CN); LIAN, Wei, Fuzhou, Fujian 350008 (CN)
(74) Representative: Plavsa, Olga
(86) International application number: PCT/CN2021/086430
(87) International publication number: WO 2021/208828

(56) References cited:
- CH-A- 345 049
- CN-U- 201 663 140
- CN-U- 202 196 769
- CN-U- 206 340 446
- CN-U- 208 722 752
- CN-U- 209 691 605
- CN-U- 211 879 241
- DE-A1- 3 345 086
- JP-A- S6 177 317
- US-A- 4 814 938
- US-A- 5 701 665
- US-A1- 2007 043 399

## Description

### TECHNICAL FIELD

The present utility model relates to a feedthrough capacitor not packaged by glass and applicable to an airtight device.

### BACKGROUND

In the prior art, a feedthrough capacitor packaged by glass is made by sintering a conductive pin and a shell with glass, then soldering a disk-like ceramic capacitor into the shell with high melting point solder, and finally sealing the disk-like ceramic capacitor with a pouring sealant. As for the product with the structure, after the disk-like ceramic capacitor is soldered, flux may be left between glass of an inner cavity and the disk-like ceramic capacitor, and is not easy to remove, thus easily affecting the electrical performance of the product.

US20070043399A1 discloses an electromagnetic interference (EMI) filter terminal for an active implantable medical device (AIMD), wherein an insulated and shielded RF telemetry pin is provided to prevent re-radiation of unwanted stray signals, including the telemetry signal itself, to adjacent sensitive circuits or lead wires. The invention provides for an EMI filter terminal assembly for an AIMD including a radio frequency (RF) telemetry pin antenna extending therethrough. The RF telemetry pin antenna includes a conductive shield extending over a portion of the RF telemetry pin antenna in non-conductive relation with the telemetry pin, and conductively connected to a ground associated with the AIMD. The assembly may also include an insulation tube between the RF telemetry pin antenna and the conductive shield covering a portion of the RF telemetry pin antenna.

### SUMMARY

The present utility model makes improvements to solve the problems in the related art, that is, the objective of the present utility model is to provide a feedthrough capacitor not packaged by glass and applicable to an airtight device according to claim 1. Further embodiments are set out in the dependent claims.

In order to achieve the above purpose, the technical solution adopted by the present utility model is as follows: a feedthrough capacitor not packaged by glass and applicable to an airtight device includes a disk-like ceramic capacitor body, a Kovar tube being disposed in an inner hole of the disk-like ceramic capacitor body, a conductive pin penetrating through an inner hole of the Kovar tube, and the conductive pin and the Kovar tube as well as the Kovar tube and the disk-like ceramic capacitor body being soldered and fixed.

Further, the conductive pin and the Kovar tube as well as the Kovar tube and the disk-like ceramic capacitor body are soldered with high melting point solder.

Further, the high melting point solder is gold-tin alloy solder.

Further, two ends of the Kovar tube extend out of the inner hole of the disk-like ceramic capacitor body.

Further, an inner electrode is provided on a wall of the inner hole of the disk-like ceramic capacitor body, and an outer electrode is provided on a peripheral side wall of the disk-like ceramic capacitor body.

Further, the disk-like ceramic capacitor body is of a multilayer laminated structure.

Compared with the prior art, the present utility model has the following effects:
(1) the present utility model has a simple and reasonable structure, the conductive pin of the feedthrough capacitor is soldered with the gold-tin solder having desirable wettability, and the distance between the conductive pin and the inner hole of the disk-like ceramic capacitor body is shortened by adding the Kovar tube between the conductive pin and the inner hole of the disk-like ceramic capacitor body, so that the amount of the gold-tin alloy solder used is effectively reduced, and the influence of thermal expansion and contraction of the gold-tin alloy solder on a disk-like ceramic capacitor can be greatly relieved, thereby improving the quality of the disk-like ceramic capacitor; and
(2) the feedthrough capacitor can be easily cleaned, with no flux left, and dense solder joints are formed to meet specific airtightness requirements.

### BRIEF DESCRIPTION OF THE DRAWINGS

FIG. 1 is a schematic diagram of a front sectional structure according to an embodiment of the present utility model.
FIG. 2 is a schematic diagram of a top sectional structure according to an embodiment of the present utility model.

In the figures:
1 denotes a disk-like ceramic capacitor body; 2 denotes an inner hole; 3 denotes a Kovar tube; 4 denotes a conductive pin; and 5 denotes gold-tin alloy solder.

### DETAILED DESCRIPTION OF THE EMBODIMENTS

Hereinafter, the present utility model will be described in further detail with reference to the accompanying drawings and detailed description.

In the description of the present utility model, it is to be understood that the terms "longitudinal", "transverse", "upper", "lower", "front", "rear", "left", "right", "vertical", "horizontal", "top", "bottom", "inner", "outer", etc. indicate orientations or positional relationships based on the orientations or positional relationships shown in the accompanying drawings, are merely for convenience in describing the present utility model, and do not indicate or imply that referenced devices or elements must have a particular orientation, be constructed and operated in a particular orientation, and thus they are not to be construed as limiting the present utility model.

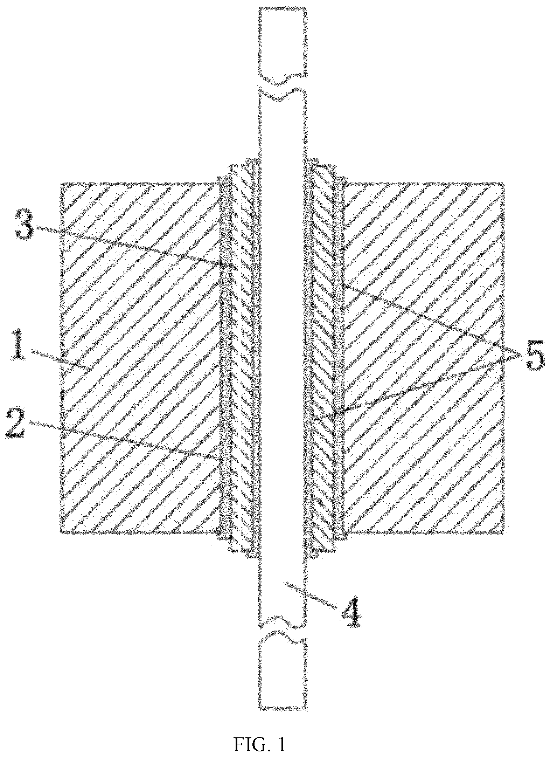

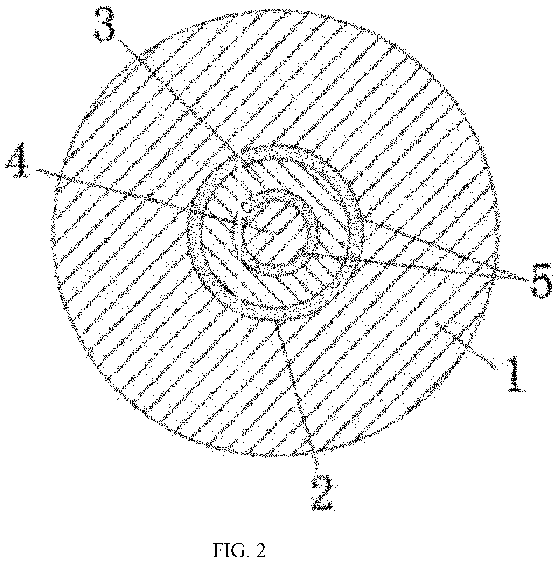

As shown in FIG. 1 and FIG. 2, a feedthrough capacitor not packaged by glass and applicable to an airtight device according to the present utility model includes a disk-like ceramic capacitor body 1 of a multilayer laminated structure. A Kovar tube 3 ("Kovar" is a registered trade mark, which is acknowledged by the applicant) is disposed in an inner hole 2 of the disk-like ceramic capacitor body 1. A conductive pin 4 penetrates through an inner hole of the Kovar tube 3. The conductive pin 4 and the Kovar tube 3 as well as the Kovar tube 3 and the inner hole 2 of the disk-like ceramic capacitor body 1 are soldered and fixed with high melting point solder, where the high melting point solder is gold-tin alloy solder 5. The distance between the conductive pin 4 and the inner hole 2 of the disk-like ceramic capacitor body 1 is shortened by adding the Kovar tube 3 between the conductive pin 4 and the disk-like ceramic capacitor body 1, so that the amount of the gold-tin alloy solder used in a soldering process is effectively reduced. Because the amount of the gold-tin alloy solder used is reduced, the influence of thermal expansion and contraction of the gold-tin alloy solder on a disk-like ceramic capacitor can be greatly relieved, thereby improving the quality of the ceramic capacitor.

In the embodiment, two ends of the Kovar tube 3 extend out of the inner hole 2 of the disk-like ceramic capacitor body 1, so as to facilitate soldering between the conductive pin and the Kovar tube. It should be noted that the two ends of the Kovar tube should extend out of the inner hole of the disk-like ceramic capacitor body by a small distance, and the specific distance may be set according to actual production needs.

In the embodiment, an inner electrode is provided on a wall of the inner hole 2 of the disk-like ceramic capacitor body 1, and an outer electrode is provided on a peripheral side wall of the disk-like ceramic capacitor body 1.

In the embodiment, the inner hole of the disk-like ceramic capacitor body is a circular hole, the Kovar tube is a circular tube, and the inner hole of the disk-like ceramic capacitor body, the Kovar tube and the conductive pin are disposed coaxially. It should be noted that the Kovar tube is of a tubular structure made of Kovar alloy material.

In the embodiment, ratios of gold and tin in the gold-tin alloy solder are 80% and 20%, respectively.

In the embodiment, during specific soldering, the conductive pin penetrates through the Kovar tube first, one end of the Kovar tube is soldered with the gold-tin alloy solder, and the gold-tin alloy solder flows downward under the action of gravity and fills a gap between the Kovar tube and the conductive pin. The Kovar tube is then disposed in the inner hole of the disk-like ceramic capacitor body, one end of the Kovar tube is soldered with the gold-tin alloy solder, and the gold-tin alloy solder flows downward under the action of gravity and fills a gap between the Kovar tube and the wall of the inner hole of the ceramic capacitor body.

The present utility model has the advantages as follows: (1) the conductive pin and the Kovar tube as well as the Kovar tube and the inner hole of the disk-like ceramic capacitor body are soldered with the gold-tin alloy solder having desirable wettability, the product can be easily cleaned, with no flux left, and dense solder joints are formed to meet specific airtightness requirements; and (2) the distance between the conductive pin and the inner hole of the disk-like ceramic capacitor body is shortened by adding the Kovar tube between the conductive pin and the inner hole of the disk-like ceramic capacitor body, so that the amount of the gold-tin alloy solder used in a soldering process is effectively reduced, and because the amount of the gold-tin alloy solder used is reduced, the influence of thermal expansion and contraction of the gold-tin alloy solder on the disk-like ceramic capacitor can be greatly relieved, thereby improving the quality of the feedthrough capacitor.

If the present utility model discloses or relates to parts or structural members that are fixedly connected to each other, unless otherwise stated, fixed connection can be understood as: removable fixed connection (such as connection using bolts or screws), or non-removable fixed connection (such as riveting and soldering). Certainly, mutual fixed connection can also be replaced by an integral structure (such as being integrally formed through a casting process), except that it is obviously impossible to use the integral forming process.

In addition, terms used to represent a positional relationship or shape used in any one of the technical solutions disclosed in the present utility model are meant to include, unless otherwise stated, states or shapes that are approximate, similar, or close thereto.

Any component provided by the present utility model may be assembled from a plurality of individual components, or may be a single component manufactured by an integral molding process.

## Claims

1. A feedthrough capacitor not packaged by glass and applicable to an airtight device, comprising a disk-like ceramic capacitor body (1), a Kovar tube (3, "Kovar" is a registered trade mark) being disposed in an inner hole (2) of the disk-like ceramic capacitor body (1), a conductive pin (4) penetrating through an inner hole (2) of the Kovar tube (3), and the conductive pin (4) and the Kovar tube (3) as well as the Kovar tube (3) and the disk-like ceramic capacitor body (1) being soldered and fixed;
**characterized in that** the conductive pin (4) and the Kovar tube (3) as well as the Kovar tube (3) and the disk-like ceramic capacitor body (1) are soldered with high melting point solder;
wherein the high melting point solder is gold-tin alloy solder (5).

2. The feedthrough capacitor not packaged by glass and applicable to an airtight device according to claim 1, wherein two ends of the Kovar tube (3) extend out of the inner hole (2) of the disk-like ceramic capacitor body (1).

3. The feedthrough capacitor not packaged by glass and applicable to an airtight device according to claim 1, wherein an inner electrode is provided on a wall of the inner hole (2) of the disk-like ceramic capacitor body (1), and an outer electrode is provided on a peripheral side wall of the ceramic capacitor body (1).

4. The feedthrough capacitor not packaged by glass and applicable to an airtight device according to claim 1 or 3, wherein the disk-like ceramic capacitor body (1) is of a multilayer laminated structure.

## Patentansprüche

1. Ein Durchführkondensator, der nicht mit Glas verpackt ist und für eine luftdichte Vorrichtung, umfassend: einen scheibenförmigen Keramikkondensatorkörper (1); ein Kovarrohr (3, "Kovar" ist eine eingetragene Marke), das in einem Innenloch (2) des scheibenförmigen Keramikkondensatorkörpers (1) angeordnet ist; einen Leitstift (4), der durch ein Innenloch (2) des Kovarrohrs (3) durchdringt, und den Leitstift (4) und das Kovarrohr (3) sowie das Kovarrohr (3) und den scheibenförmigen Keramikkondensatorkörper (1) gelötet und befestigt ist;
**dadurch gekennzeichnet, dass** der Leitstift (4) und das Kovarrohr (3) sowie das Kovarrohr (3) und der scheibenartige Keramikkondensatorkörper (1) mit Hochschmelzpunktlöt gelötet sind;
wobei das Hochschmelzpunktlöt Gold-Zinnlegierungslöt (5) ist.

2. Der Durchführungskondensator nach Anspruch 1, dass sich zwei Enden des Kovarrohrs (3) aus dem Innenloch (2) des scheibenförmigen Keramikkondensatorkörpers (1) erstrecken.

3. Der Durchführungskondensator nach Anspruch 1, dass an einer Wand des inneren Lochs (2) des scheibenförmigen Keramikkondensatorkörpers (1) eine Innenelektrode und an einer umlaufenden Seitenwand des Keramikkondensatorkörpers (1) eine Außenelektrode vorgesehen sind.

4. Der Durchführkondensator, nach einem der Ansprüche 1 oder 3, dass der scheibenartige Keramikkondensatorkörper (1) aus einer mehrschichtigen laminierten Struktur besteht.

## Revendications

1. Un condensateur de traversée non encapsulé par du verre et applicable à un dispositif étanche à l'air, comprenant un corps de condensateur en céramique à disque (1), un tube de Kovar (3, « Kovar » est une marque déposée) disposé dans un trou intérieur (2) du corps de condensateur en céramique à disque (1), une broche conductrice (4) pénétrant dans un trou intérieur (2) du tube de Kovar (3), et la broche conductrice (4) et le tube de Kovar (3) ainsi que le tube de Kovar (3) et le corps de condensateur en céramique à disque (1) étant soudés et fixés;
**caractérisé en ce que** la broche conductrice (4) et le tube de Kovar (3) ainsi que le tube de Kovar (3) et le corps de condensateur en céramique à disque (1) sont soudés par soudure à haut point de fusion ;
dans lequel la soudure à haut point de fusion est une soudure en alliage or-étain (5).

2. Le condensateur de traversée selon la revendication 1, dans lequel deux extrémités du tube de Kovar (3) s'étendent à l'extérieur du trou intérieur (2) du corps de condensateur en céramique à disque (1).

3. Le condensateur de traversée selon la revendication 1, dans lequel une électrode interne est prévue sur une paroi du trou interne (2) du corps de condensateur en céramique à disque (1), et une électrode externe est prévue sur une paroi latérale périphérique du corps de condensateur en céramique (1).

4. Le condensateur de traversée selon la revendication 1 ou 3, dans lequel le corps de condensateur en céramique à disque (1) est de structure stratifiée multicouche.
